# EUROPEAN PATENT APPLICATION

(11) **EP 2 638 883 A1**
(43) Date of publication of application: **18.09.2013**
(21) Application number: 10859489.6
(22) Date of filing: 12.11.2010
(51) Int. Cl.: A61F 2/92, A61F 2/90, A61L 31/06

(54) **SLIDE FASTENER BIOABSORBABLE STENT AND APPLICATION THEREOF**

(71) Applicant: Xin Hua Hospital Affiliated To Shanghai Jiao Tong University School of Medicine, Shanghai 200092 (CN)
(72) Inventor: SUN, Kun, Shanghai 200092 (CN); SUN, Kang, Shanghai 200127 (CN); FENG, Qimao, Shanghai 200092 (CN)
(74) Representative: Wilson, Justin Scott
(86) International application number: PCT/CN2010/078664
(87) International publication number: WO 2012/061992

(57) **Abstract**

A slide fastener bioabsorbable stent is applied as a cardiovascular system stent or a lumen stent in the disease of cardiovascular or narrow lumen. The slide fastener bioabsorbable stent includes a snap fastener stent, an edge slide fastener stent, a middle slide fastener stent, and a double fastener stent. The slide fastener bioabsorbable stent has good degradability and biocompatibility, and is more suitably used as a pediatric vascular stent, with which no late-onset stent thromboses after implanted, and thus it is not necessary to long-term take antiplatelet drugs and the subsequent surgical operation may not be affected. Also, it has a strong support, and thus can be widely used as a cardiovascular system stent or a lumen stent in the disease of cardiovascular or narrow lumen. The slide fastener bioabsorbable stent has simple production and convenient drug carrying, which can be used as a carrier of medicine or gene treatment. The stent can be matched with a delivery system, hence the difficulty of the surgery operation is reduced. Many animal experiments show that slide fastener bioabsorbable stent has high success ratio when being used, and has apparent curative effect and good clinical application prospect.

## Description

### TECHNICAL FIELD

The present invention relates to a medical instrument, specifically to a slide fastener bioabsorbable stent and application thereof.

### BACKGROUND ART

Congenital heart disease (abbreviation CHD) is the most common cardiovascular disease in the pediatric period, the morbidity rate of live births is 0.678%. The infant CHD of congenital pulmonary artery and pulmonary vein stenosis, cardinal vein and aorta and their branches stenosis take up 7%-15% of all CHD. Moreover, the quantity of patients who suffer from postoperative right ventricle-pulmonary artery artificial pipeline (RV-PA pipeline) stenosis, postoperative pulmonary artery and pulmonary vein restenosis, cardinal vein and aorta and their branches restenosis and Fontan channel stenosis, will increase along with the improvement of CHD surgery operation ability.

For the diseases of infant congenital or postoperative pulmonary artery and pulmonary artery branches stenosis and aorta coarctation, both methods of balloon angioplasty and vascular stent implantation are secure intervention treatment, but the complication rate of the former is higher, especially for small infants, so the stent implantation is considered to be a better choice. In consideration of the specific physiological characteristics of small infants, the ideal pediatric vascular stent should have safety, efficiency, degradability, small initial diameter and transmissibility. Stent safety means good blood compatibility (without thrombosis, hemolysis, etc.), histocompatibility (high purity, no toxicity, no stimulation, no carcinogenicity, no mutagenicity, no antigenicity). Secondly, stent efficiency means the stent plays a supportive role in blood vessel, and the stent should have sufficient radial strength. Because the infant blood vessels are in growth and development stage, the stent should have degradability that facilitates the further growth and development of blood vessels. At the same time, the stent requires small initial diameter because of the smaller blood vessels of the infants; transmissibility and X-radiopaque property make the stent easily to be delivered to the stenosis position.

The stent plays a supportive role in blood vessel to prevent retraction in the early days; actually, due to the stent endothelialization and the late blood vessel wall reconstruction, the stent just plays a temporary supportive role. The common vascular stent is weaved by metal at present. The fixed size of metallic stent does not change with the growth of blood vessel after implanted, and that easily leads to the mismatch with stent and blood vessel size and factitious stenosis at later stage, especially, the metallic stent is not suitable to using for pediatric vascular stent which is used for the children with growing characteristics.

The metallic stent also displays the following disadvantages: (1) easy to thrombosis and need long-term antiplatelet therapy; (2) life-long retention in human body, impact subsequent possible surgical treatment; (3) appear artifact in nuclear magnetic resonance and CT examination; (4) may change the geometric configuration of blood vessel to bring about branches blockage; (5) obstruct subsequent lumen reconstruction and expansion; (6) there will be a residual small gap after implanted if the metallic stent is not matched with the blood vessel wall. Therefore, if a bioabsorbable stent, that the performance of said bioabsorbable stent is similar to the metallic stent and said bioabsorbable stent can be completely absorbed after finishing its mission, is researched and developed, and the disadvantages of metallic stent can be overcome, that will open up a new horizon for CHD intervention treatment.

That the bioabsorbable stent is used for treating cardiovascular disease has been studied at home and abroad. Nowadays the major materials of bioabsorbable stent are poly-L-lactic acid, polydioxanone and polycaprolactone, and these materials have been passed the U.S.A FDA standard and can be implanted in the body. The bioabsorbable stent can be designed as Igaki-Tamai stent, REVA stent and four-leaf structure stent, the common characteristic of these stents is that they are complete cylinders before expansion. But these stents can not be widely applied in the disease of narrow lumen except blood vessel stenosis because of their insufficient supporting force, easy to elastic retraction, complicated manufacturing process, high cost and existing restriction by supporting force.

China Application of publication No. CN101484195A discloses a composite stent, this invention discloses a biodegradable or bioabsorbable multiple-layered or composite stent which includes a bioabsorbable ceramic material coated with a biodegradable polymeric material. But a slide fastener bioabsorbable stent with strong support and convenient manufacture and usage, and it is applied as a cardiovascular system stent or a lumen stent in the disease of cardiovascular or narrow lumen, have not been reported.

### SUMMARY OF THE INVENTION

The purpose of this invention is aim at the drawbacks of the prior art and to provide the use of a slide fastener bioabsorbable stent.

Another purpose of this invention is to provide a double fastener stent. To achieve the above first purpose, this invention takes the following technical solutions:
use of a slide fastener bioabsorbable stent as a cardiovascular system stent or a lumen stent in the disease of cardiovascular or narrow lumen.

Said disease of narrow cardiovascular system is coronary artery stenosis, carotid artery stenosis, renal artery stenosis, pulmonary artery and its branches stenosis, aorta and its branches stenosis, cardinal vein stenosis or pulmonary vein stenosis.

Said disease of narrow lumen is trachea stenosis, esophagus stenosis, biliary tract stenosis, urethra stenosis or intestinal tract stenosis.

Said stent comprising:
an applanate stent body with mesh structure,
a stent head, which is located at one end of said stent body, said stent head is integrated with said stent body with matched size, and which provides a slide fastener effect when the stent is being curled,
stent clips, which are integrated with said stent body and fix the tubular structure of the stent when it is being curled.

Said stent material is polydioxanone (PDO), polylactic acid (PLA), polycaprolactone (PCL), polyglycolic acid (PGA) or polyhydroxybutyrate (PHB) macromolecular polymer.

Said stent material is polydioxanone (PDO).

Said stent also includes a delivery device, said delivery device comprising:
an outer cannula, consisting of a proximal end, a distal end and the cavity among the two ends, and
an inner sheath, consisting of a proximal end, a distal end and the cavity among the two ends, the external diameter of said inner sheath is proper to insert in the cavity of said outer cannula, and
a balloon catheter, consisting of a proximal end, a distal end and the cavity among the two ends, the external diameter of said balloon catheter is proper to insert in the cavity of said inner sheath, there is a balloon in the distal end of said balloon catheter, the lamellar slide fastener stent can be coiled on the surface of said balloon and subsequently delivered to the narrow lumen through the catheter.

To achieve the above second purpose, this invention takes the following technical solutions:
A new bioabsorbable stent, said stent includes a snap fastener stent, an edge slide fastener stent, a middle slide fastener stent and a double fastener stent, wherein said double fastener stent comprising:
   an applanate stent body with mesh structure,
   a stent head, which is located at one end of said stent body, said stent head is integrated with said stent body with matched size, and which provides a slide fastener effect when the stent is being curled,
   stent clips, which are integrated with said stent body and consist of barbs located at the two sides of said stent body and stent head clips located at said stent head, and which fix the tubular structure of the stent when it is being curled.

The advantages of this invention are:
1. The slide fastener bioabsorbable stent has good degradability and biocompatibility, and is more suitably used as a pediatric vascular stent, with which no late-onset stent thromboses after implanted, and thus it is not necessary to long-term take antiplatelet drugs and the subsequent surgical operation may not be affected.
2. The slide fastener bioabsorbable stent has a strong support, and thus can be widely used as a cardiovascular system stent or a lumen stent in the disease of cardiovascular or narrow lumen.
3. The slide fastener bioabsorbable stent has simple production and convenient drug carrying, which can be used as a carrier of medicine or gene treatment.
4. The stent can be matched with a delivery system, hence the difficulty of the surgery operation is reduced.
5. Many animal experiments show that slide fastener bioabsorbable stent has high success ratio when being used, and has apparent curative effect and good clinical application prospect.

### BRIEF DESCRIPTION OF THE DRAWINGS

Fig. 1 is a schematic view of a snap fastener stent of the slide fastener bioabsorbable stent.
Fig. 2 is a schematic view of an edge slide fastener stent of the slide fastener bioabsorbable stent.
Fig. 3 is a schematic view of a middle slide fastener stent of the slide fastener bioabsorbable stent.
Fig. 4 is a schematic view of a double fastener stent of the slide fastener bioabsorbable stent.
Fig. 5 is a schematic view of a delivery system of the slide fastener bioabsorbable stent.

### DETAILED DESCRIPTION OF THE PREFERRED EMBODIMENTS

The invention is further described by combining with embodiments.

The embodiments of the invention are further described in detail by combining with figures.

The relevant signs and components of the figures are listed as follows:

| | |
|---|---|
| 1. stent body | 2. stent head |
| 11. meshes | 12. barbs |
| 21. framework | 22. stent clips |
| 23. clip structure | 3. outer cannula |
| 4. inner sheath | 5. balloon catheter |
| 31. Y-type adapter | 41. Y-type adapter |
| 51. balloon | 52. cone |

### Example 1 structural design and comparison of the bioabsorbable

### stents

According to the structure of present clinical vascular stent and the properties of bioabsorbable material, three structures of stents are designed by PDO material.
(1) Self-expandable net-tube stent: the net-tube stent is made by using a stainless steel cylindrical mold. The diameter of the mold is matched with that of the stent, circular holes are made evenly on the circumference of two ends of the mold, and steel needles are inserted into said circular holes with the same number at two ends and mutually aligned. PDO fiber are weaved back and forth on the mold with paying attention to the weaving order, the cylinder is gotten directly with mutual interlaced and restrictive fiber, and then heat setting (90 °C, 4 hours) to maintain this shape, the weave density and inclined angle of fibers can be adjusted.
(2) Self-expandable Zigzag stent: the Zigzag stent is made by macromolecular fiber with heat setting method. Pinholes are made by wire-electrode cutting method on a steel plate of 3 mm thickness, and steel needles are inserted into said pinholes. The PDO fiber is fixed to sine wave shape by steel needles then placed under appropriate setting conditions, so that the fiber has a Zigzag shape with the shape memory effect. Several fibers are conglutinated together to form a cylindrical stent according to the needed diameter of stent.
(3) Slide fastener stent: the slide fastener stent is fabricated by a three-dimensional micro-jetting free molding technology. The stent is a lamellar structure before implanted and consists of a stent body with mesh structure, a stent head with slide fastener effect and stent clips. The stent is coiled on a balloon, and one end of the stent is inserted into another end with specially designed lock catch (similar to "belt buckle") to form a tubular stent. The tubular stent is dilated with the balloon expansion and buckled when the balloon is withdrawn, the stent can not slide to inside and maintain supporting effect. Meanwhile, an X-radiopaque metallic marker is mounted in the mesh structure of the slide fastener stent in order to mark the stent under X-ray.

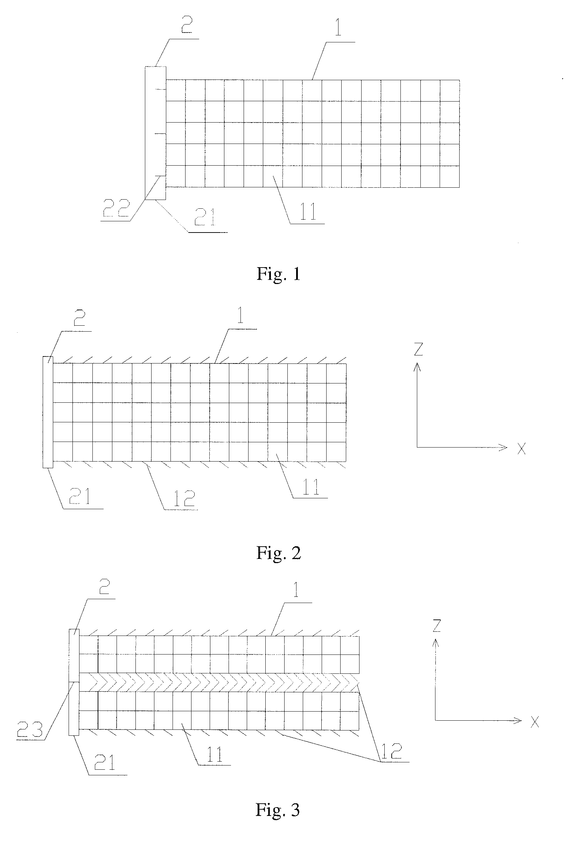

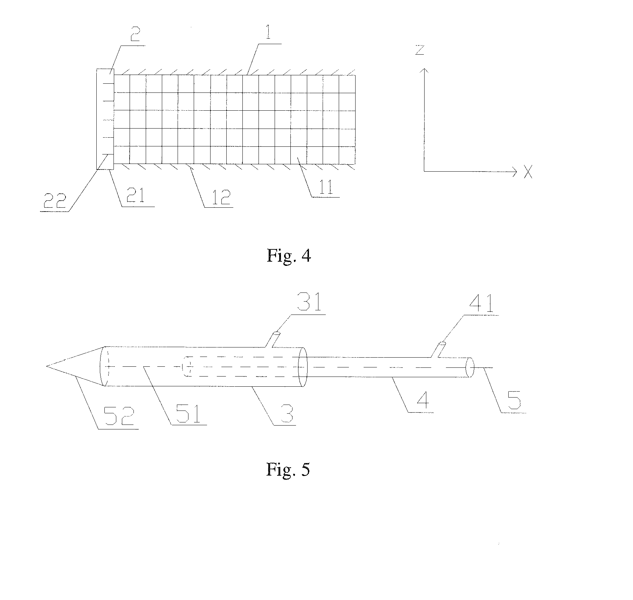

The slide fastener stent can be divided into a snap fastener type, an edge slide fastener type, a middle slide fastener type and a double fastener type in according to the position of the slide fastener.
□ The snap fastener type: as shown in Fig. 1, it shows a slide fastener bioabsorbable stent in an embodiment. The stent consists of a lamellar stent body (1) and a stent head (2) located in one end of said stent body (1). The stent head (2) consists of stent clips (22) and a framework (21); there are rows of meshes (11) with the same or different size in stent body (1). In an embodiment of this invention, the size of each mesh (11) is even-distributed, for example, the size of meshes (11) is 0.5-3 mm. The meshes (11) can be any shape, including circle, ellipse, square, rectangle, triangle, polygon, etc. In an embodiment of this invention, the meshes (11) are circle. The stent clips (22) are located near the stent head (2) and contain 2-4 protruding clips; these protruding clips and the framework (21) together form the slide fastener apparatus of this invention. There also can be more protruding clips depending on the required size and application of the stent. The length of the protruding clip is 0.5-1 mm, and the protruding clip is at an angle usually 20-40 degrees relative to the stent plane. But technicians should understand in their field that the degrees can be other value. The size of the framework (21) is matched with the stent body (1), so that the inner protruding clips could slide strictly along the direction of sheet length and avoid dislocation in stent curly process. The said protruding clips can be inserted into any row of meshes (11) to lead to the lamellar stent form to a tubular stent in sliding process.
□ The edge slide fastener type: as shown in Fig. 2, it shows a slide fastener bioabsorbable stent in another embodiment. The stent consists of a lamellar stent body (1) and a stent head (2) located in one end of said stent body (1). The stent head (2) includes a framework (21) which the size matched with the stent body (1). There are several barbs (12) on the two sides of the stent body (1). The stent includes an extended longitudinal axis Z parallel to the stent head (2) and an extended lateral axis X perpendicular to the stent head (2) as shown in Fig. 2. There are rows of meshes (11) with the same or different size in the stent body (1). In an embodiment of this invention, the size of each mesh (11) is even-distributed, for example, the size of meshes (11) is 0.5-3 mm. The meshes (11) can be any shape, including circle, ellipse, square, rectangle, triangle, polygon, etc. In an embodiment of this invention, the meshes (11) are circle. In the embodiment as shown in Fig. 2, the stent clips are showed as the barbs (12) located on the two sides of the stent body (1), said stent body (1) could pass through said framework (21) in stent curly process, and the barbs (12) could slide along the two edges of said framework (21). Said barbs (12) could buckle said framework (21) in sliding process, thus the lamellar stent is fixed as a tubular stent. In an embodiment, the size of barbs (12) is 0.1 mm. All of barbs (12) extend away from the stent head (2) and make an angle relative to the lateral axis of the stent, for instance, 30-60 degrees. In an embodiment of this invention, the barbs (12) make a 30 degrees angle relative to the lateral axis of the stent.
□ The middle slide fastener type: as shown in Fig. 3, it shows a slide fastener bioabsorbable stent in another embodiment. The stent consists of a lamellar stent body (1) and a stent head (2) located in one end of said stent body (1). The stent head (2) consists of a framework (21) and a clip structure (23) located in the middle of said framework (21), the two ends of said clip structure (23) connect with said framework (21). The stent body (1) includes barbs (12) which correspond to the clip structure (23), these barbs (12) extend away from the stent head (2). As shown in Fig. 3, the stent includes an extended longitudinal axis Z parallel to the stent head (2) and an extended lateral axis X perpendicular to the stent head (2). There are rows of meshes (11) with the same or different size in the stent body (1). In an embodiment of this invention, the size of each mesh (11) is even-distributed, for example, the size of meshes (11) is 0.5-3 mm. The meshes (11) can be any shape, including circle, ellipse, square, rectangle, triangle, polygon, etc. In an embodiment of this invention, the meshes (11) are circle. In an embodiment, the length of said clip structure (23) located in the stent head (2) is 0.5-1 mm. In an embodiment, the size of said barbs (12) located in the stent body (1) is 0.1 mm. The said barbs (12) make an angle relative to the lateral axis of the stent, for instance, 30-60 degrees. In an embodiment of this invention, the barbs (12) make a 30 degrees angle relative to the lateral axis of the stent. The said barbs (12) slide along two sides of the clip structure (23) located in the said framework (21) of the stent head (2) in stent curly process, said barbs (12) could buckle the clip structure (23) located in the said framework (21) in sliding process, thus the lamellar stent is fixed as a tubular stent.
□ The double fastener type: as shown in Fig. 4, it shows a slide fastener bioabsorbable stent in an embodiment. The stent consists of a lamellar stent body (1) and a stent head (2) located in one end of said stent body (1). The stent head (2) consists of stent clips (22) and a framework (21); there are rows of meshes (11) with the same or different size in stent body (1); there are several barbs (12) on the two sides of the stent body (1). The stent clips (22) are located near the stent head (2) and contain 2-4 protruding clips; these protruding clips and the framework (21) together form the slide fastener apparatus of this invention. There also can be more protruding clips depending on the required size and application of the stent. The length of the protruding clip is 0.5-1 mm, and the protruding clip is at an angle usually 20-40 degrees relative to the stent plane. But technicians should understand in their field that the degrees can be other value. The size of the framework (21) is matched with the stent body (1), so that the inner protruding clips could slide strictly along the direction of sheet length and avoid dislocation in stent curly process. The said protruding clips can be inserted into any row of meshes (11) to lead to the lamellar stent form to a tubular stent in stent sliding process. The said stent body (1) could pass through said framework (21) in stent curly process, and the barbs (12) could slide along the two edges of said framework (21). Said barbs (12) could buckle said framework (21) in sliding process, thus the lamellar stent is fixed as a tubular stent. In an embodiment, the size of barbs (12) is 0.1 mm. All of barbs (12) extend away from the stent head (2) and make an angle relative to the lateral axis of the stent, for instance, 30-60 degrees. In an embodiment of this invention, the barbs (12) make a 30 degrees angle relative to the lateral axis of the stent. The double fastener stent includes both stent clips (22) and barbs (12), so it has stronger support and can ensure the stent buckling in its employment.

The embodiment as shown in Fig. 2, Fig. 3 and Fig. 4, all of barbs (12) are in the same direction, the stent can not be retracted after buckled. In the procedure of delivered toward to the body, the stent is rolled up and tightly attached to the balloon (51) of the delivery device. When the delivery device arrive at the designated site, the balloon (51) is dilated to expand the diameter of the stent, then the balloon (51) is sucked back and the stent is buckled as a result of the pressure of the blood vessel wall to play a supportive role.

### 1. Determination of the mechanical properties of the stent

Ten stents are selected with different structure, different thickness and different diameter, respectively. Determination contents include: radial strength, stent surface coverage rate, stent axial contraction rate, stent expansion rate.

The results of the mechanical properties of the stent are shown in table 1. Self-expandable net-tube stents and Zigzag stents fail to meet clinical requirement because of their lower radial strength (usually 80-120 Kpa) and are not suitable for the experiment. The radial strengths of four kinds of balloon expandable slide fastener stents are higher than 80 Kpa and reach that of the metallic stents. Meanwhile the axial contraction rates of the slide fastener stents are all 0% and superior to that of the metallic stents (5%); but expansion rates of the slide fastener stents (29%) are slightly inferior to that of the metallic stents (25%); the surface coverage rates of the slide fastener stents are obviously higher than that of the metallic stents (20%).

**Table 1 The results of mechanical properties of the stents of 0.3 mm thickness with different structures**

| | n | Diameter (mm) | Length (mm) | Radial strength (Kpa) | Surface coverage rate (%) | Axial contraction rate (%) | Expansion rate (%) |
|---|---|---|---|---|---|---|---|
| Stainless steel stent | 5 | 8 | 20 | 135±5.80 | 21±2.00 | 5±1.20 | 22±3.00 |
| Zigzag stent | 10 | 8 | 20 | 22.5±3.70 | 26±3.00 | 2±0.30 | 20±2.00 |
| Net-tube stent | 10 | 8 | 20 | 32±4.30 | 30±3.00 | 3±0.50 | 25±3.00 |
| Snap fastener stent | 10 | 8 | 20 | 110.8±16.90 | 24±3.00 | 0 | 30±3.00 |
| Middle slide fastener stent | 10 | 8 | 20 | 125.1±15.70 | 39±4.00 | 0 | 29±2.00 |
| Edge slide fastener stent | 10 | 8 | 20 | 139.6±15.50 | 40±4.00 | 0 | 29.5±2.00 |
| Double fastener stent | 10 | 8 | 20 | 135.5±15.50 | 31±4.00 | 0 | 30±3.00 |

The results of the radial strengths of the edge slide fastener PDO stents with different thickness and different diameter are shown in table 2. The results show that: for the stents with the same thickness, the radial strengths are decreased gradually with increasing the diameter; for the stents with the same diameter, the radial strengths are increased gradually with increasing the thickness; the thickness of 0.20 mm can meet the radial strength requirement of the diameter of 4-8 mm.

**Table 2 The results of the radial strengths of the edge slide fastener PRO stents with different thickness and different diameter (Kpa)**

| | 4 mm n=5 | 6 mm n=5 | 8 mm n=5 | 10 mm n=5 |
|---|---|---|---|---|
| 0.20mm | 163±18.50 | 153±14.60 | 107±14.30 | 81±7.50 |
| 0.25mm | 187±21.70 | 168±16.50 | 125±15.60 | 104±14.20 |
| 0.30mm | 211±30.20 | 182±22.60 | 159.6±16.50 | 123±14.70 |

### 2. Simulation in vitro

①The diameter of selected simulative artificial blood vessel is 6 mm, the ratio of stent diameter and blood vessel diameter is 1.3:1. The balloon of the delivery system is sucked into negative pressure by the force pump; four kinds of slide fastener stents are respectively coiled on the surfaces of the balloons when the outer cannulas are withdrawn, then the stents are enclosed when the outer cannulas are pushed to the cone portion. The delivery system is delivered to the artificial blood vessel and the stent is dilated and released at 10 atmospheres for 30 s.

②Performance index

Acute elastic recoil rate: the acute elastic recoil rate = (the diameter of the fully expansive stent - the diameter of stent after the balloon is withdrawn) / the diameter of the fully expansive stent x100%.

Successful locking ratio: The evaluation criteria are: success, the stent clips are locked after the balloon is withdrawn, the stent play a supportive role; failure, the stent clips are not locked after the balloon is withdrawn, the stent head curl inward and fail to support the blood vessel.

The simulated results in vitro of four kinds of slide fastener stents are shown in table 3. All of the middle slide fastener stents, the edge slide fastener stents and the double fastener stents are locked successfully to support the blood vessel; there is one failing case of the snap fastener stent. Four kinds of slide fastener stents have tiny acute elastic recoil rate (0.40±0.10%).

**Table 3 The simulated results in vitro of four kinds of slide fastener stents**

| | n | Releasing pressure (atm) | Successful locking ratio (%) | Acute elastic recoil rate (%) |
|---|---|---|---|---|
| Snap fastener stent | 10 | 8±2.00 | 90 | 0.50±0.10 |
| Middle slide fastener stent | 10 | 10±2.00 | 100 | 0.40±0.10 |
| Edge slide fastener stent | 10 | 10±2.00 | 100 | 0.30±0.05 |
| Double fastener stent | 10 | 10±2.00 | 100 | 0.2510±0.05 |

### 3. Conclusions

The test results of the mechanical properties of the PDO stents confirm that: the radial strengths of the self-expandable net-tube stent and self-expandable Zigzag stent could not meet requirement, but the radial strengths of four kinds of slide fastener stents could meet the clinical requirement; that the edge slide fastener stent and the middle slide fastener stent could lock successfully prove the feasibility of these designs; four kinds of slide fastener stents have the advantages of low acute elastic recoil rate, zero axial contraction rate, good tracer ability, etc; but good radial strength is depended on the largish surface coverage rate, and the expansivity of the slide fastener stent is slightly inferior to that of the metallic stent.

### Example 2 the releases of four kinds of slide fastener PDO stents in vitro

### 1. The release of snap fastener polydioxanone (PDO) stent in vitro

### 1.1 Materials and methods:

Materials: ten snap fastener polydioxanone (PDO) stents of 20x8 mm, rubber hose of 6 cm diameter, stent delivery system and force pump.

### Methods:

(1) The balloon of the delivery system is sucked into negative pressure by the force pump; the stent is coiled on the surface of the balloon when the outer cannula is withdrawn, then the stent is enclosed when the outer cannula is pushed to the cone portion.
(2) The stent delivery system is delivered to the artificial blood vessel target by following a guide wire fed; the stent is dilated and released at 12 atmospheres for 30 s.
(3) The balloon is sucked into negative pressure and after that the balloon is withdrawn.

### 1.2 Performance index

(1) The diameter of blood vessel lumen: to measure the diameter of blood vessel lumen by stent expansion after the balloon is withdrawn.
(2) Acute elastic recoil rate: the acute elastic recoil rate of stent = (the diameter of the fully expansive stent - the diameter of stent after the balloon is withdrawn) / the diameter of the fully expansive stent x100%.
(3) Successful locking ratio: the evaluation criteria are: success, the stent clips are locked after the balloon is withdrawn; failure, the stent clips are not locked after the balloon is withdrawn, the stent head curl inward.

### 1.3 Results

The entire snap fastener PDO stents could release under the common pressure (10-14 atm) and lock successfully, and no stent curl toward intracavity; the diameter of stent is basically maintained at the predetermined diameter of lumen, the stents have very low acute elastic recoil rate (0.5%). The results prove that the stent design is feasible.

**Table 4 The results of the snap fastener PDO stents**

| | n | Releasing pressure (atm) | Diameter of lumen by stent expansion (mm) | Acute elastic recoil rate (%) | Successful locking ratio (%) |
|---|---|---|---|---|---|
| Snap fastener stent | 10 | 12±2 | 7.79±0.2 | 0.5±0.1 | 100 |

### 2. The release of edge slide fastener polydioxanone (PDO) stent in vitro

### 2.1 Materials and methods:

Materials: ten edge slide fastener polydioxanone (PDO) stents of 20x8 mm, rubber hose of 6 cm diameter, stent delivery system and force pump.

Methods: see also the release of snap fastener polydioxanone (PDO) stent in vitro.

### 2.2 Performance index

See also the release of snap fastener polydioxanone (PDO) stent in vitro.

### 2.3 Results

The entire edge slide fastener PDO stents could release under the common pressure (10-14 atm) and lock successfully, and no stent curl toward intracavity; the diameter of stent is basically maintained at the predetermined diameter of lumen, the stents have very low acute elastic recoil rate (0.3%). The results prove that the stent design is feasible.

**Table 5 The results of the edge slide fastener PDO stents**

| | n | Releasing pressure (atm) | Diameter of lumen by stent expansion (mm) | Acute elastic recoil rate (%) | Successful locking ratio (%) |
|---|---|---|---|---|---|
| Edge slide fastener stent | 10 | 12±2 | 7.82±0.2 | 0.3±0.1 | 100 |

### 3. The release of middle slide fastener polydioxanone (PDO) stent in vitro

### 3.1 Materials and methods:

Materials: ten middle slide fastener polydioxanone (PDO) stents of 20×8 mm, rubber hose of 6 cm diameter, stent delivery system and force pump.

Methods: see also the release of snap fastener polydioxanone (PDO) stent in vitro.

### 3.2 Performance index

See also the release of snap fastener polydioxanone (PDO) stent in vitro.

### 3.3 Results

The entire middle slide fastener PDO stents could release under the common pressure (10-16 atm) and lock successfully, and no stent curl toward intracavity; the diameter of stent is basically maintained at the predetermined diameter of lumen, the stents have very low acute elastic recoil rate (0.43%). The results prove that the stent design is feasible.

**Table 6 The results of the middle slide fastener PDO stents**

| | n | Releasing pressure (atm) | Diameter of lumen by stent expansion (mm) | Acute elastic recoil rate (%) | Successful locking ratio (%) |
|---|---|---|---|---|---|
| Middle slide fastener stent | 10 | 13±3 | 7.83±0.2 | 0.43±0.1 | 100 |

### 4. The release of double fastener polydioxanone (PDO) stent in vitro

### 4.1 Materials and methods:

Materials: ten double fastener polydioxanone (PDO) stents of 20x8 mm, rubber hose of 6 cm diameter, stent delivery system and force pump. Methods: see also the release of snap fastener polydioxanone (PDO) stent in vitro.

### 4.2 Performance index

See also the release of snap fastener polydioxanone (PDO) stent in vitro.

### 4.3 Results

The entire double fastener PRO stents could release under the common pressure (10-14 atm) and lock successfully, and no stent curl toward intracavity; the diameter of stent is basically maintained at the predetermined diameter of lumen, the stents have very low acute elastic recoil rate (0.3%). The results prove that the stent design is feasible.

**Table 7 The results of the double fastener PRO stents**

| | n | Releasing pressure (atm) | Diameter of lumen by stent expansion (mm) | Acute elastic recoil rate (%) | Successful locking ratio (%) |
|---|---|---|---|---|---|
| Double fastener stent | 10 | 12±2 | 7.67±0.3 | 0.25±0.1 | 100 |

### 5. Conclusions

The releases of four kinds of slide fastener PRO stents in vitro are examined, the results prove that: four kinds of slide fastener PRO stents could release under the common pressure and lock successfully, and no stent curl toward intracavity; the diameters of stents are basically maintained at the predetermined diameter of lumen, the stents have very low acute elastic recoil rate. The results demonstrate the stent designs are feasible. The clips of the snap fastener stent must have a certain angle in order to ensure be buckled by the meshes, of cause, the risk of failure exist too, the design need to be improved. The design of edge slide fastener stent makes up the drawback of snap fastener stent, which can ensure stent is buckled, but the barbs of edge slide fastener stent must be designed elaborately and no influence on stent expansion; and the length of edge slide fastener stent depend upon the requirement of maximum supporting force, the drawback of the design is not suitable for long stent. The design of middle slide fastener stent makes up the drawback of edge slide fastener stent, the middle slide fastener stent equivalently combines snap fastener stent and edge slide fastener stent so that it includes edge slide fastener and middle slide fastener and is a suitable stent for long lesion. The double fastener stent includes both stent clips and barbs, so it has a stronger support and can ensure the stent is buckled firmly in employment.

### Example 3 the releases of five kinds of materials of edge slide fastener stents in vitro

### 1. The release of edge slide fastener polydioxanone (PDO) stent in vitro

### 1.1 Materials and methods:

Materials: ten edge slide fastener polydioxanone (PDO) stents of 20x8 mm, rubber hose of 6 cm diameter, stent delivery system and force pump.

Methods: see also the release of snap fastener polydioxanone (PDO) stent in vitro.

### 1.2 Performance index

See also the release of snap fastener polydioxanone (PDO) stent in vitro.

### 1.3 Results

The entire edge slide fastener PDO stents could release under the common pressure (10-14 atm) and lock successfully, and no stent curl toward intracavity; the diameter of stent is basically maintained at the predetermined diameter of lumen, the stents have very low acute elastic recoil rate (0.3%). The results prove that the stent design is feasible.

**Table 8 The results of the edge slide fastener PRO stents**

| | n | Releasing pressure (atm) | Diameter of lumen by stent expansion (mm) | Acute elastic recoil rate (%) | Successful locking ratio (%) |
|---|---|---|---|---|---|
| Edge slide fastener stent | 10 | 12±2 | 7.82±0.2 | 0.3±0.1 | 100 |

### 2. The release of edge slide fastener polycaprolactone (PCL) stent in vitro

### 2.1 Materials and methods:

Materials: ten edge slide fastener polycaprolactone (PCL) stents of 20x8 mm, rubber hose of 6 cm diameter, stent delivery system and force pump.

Methods: see also the release of snap fastener polydioxanone (PDO) stent in vitro.

### 2.2 Performance index

See also the release of snap fastener polydioxanone (PDO) stent in vitro.

### 2.3 Results

The entire edge slide fastener PCL stents could release under the common pressure (11-15 atm) and lock successfully, and no stent curl toward intracavity; the diameter of stent is basically maintained at the predetermined diameter of lumen, the stents have very low acute elastic recoil rate (0.35%). The results prove that the stent design is feasible.

**Table 9 The results of the edge slide fastener PCL stents**

| | n | Releasing pressure (atm) | Diameter of lumen by stent expansion (mm) | Acute elastic recoil rate (%) | Successful locking ratio (%) |
|---|---|---|---|---|---|
| Edge slide fastener stent | 10 | 13±2 | 7.82±0.2 | 0.35±0.1 | 100 |

### 3. The release of edge slide fastener polyglycolic acid (PGA) stent in vitro

### 3.1 Materials and methods:

Materials: ten edge slide fastener polyglycolic acid (PGA) stents of 20x8 mm, rubber hose of 6 cm diameter, stent delivery system and force pump.

Methods: see also the release of snap fastener polydioxanone (PDO) stent in vitro.

### 3.2 Performance index

See also the release of snap fastener polydioxanone (PDO) stent in vitro.

### 3.3 Results

The entire edge slide fastener PGA stents could release under the common pressure (11-15 atm) and lock successfully, and no stent curl toward intracavity; the diameter of stent is basically maintained at the predetermined diameter of lumen, the stents have very low acute elastic recoil rate (0.35%). The results prove that the stent design is feasible.

**Table 10 The results of the edge slide fastener PGA stents**

| | n | Releasing pressure (atm) | Diameter of lumen by stent expansion (mm) | Acute elastic recoil rate (%) | Successful locking ratio (%) |
|---|---|---|---|---|---|
| Edge slide fastener stent | 10 | 13±2 | 7.82±0.2 | 0.35±0.1 | 100 |

### 4. The release of edge slide fastener polyhydroxybutyrate (PHB) stent in vitro

### 4.1 Materials and methods:

Materials: ten edge slide fastener polyhydroxybutyrate (PHB) stents of 20x8 mm, rubber hose of 6 cm diameter, stent delivery system and force pump.

Methods: see also the release of snap fastener polydioxanone (PDO) stent in vitro.

### 4.2 Performance index

See also the release of snap fastener polydioxanone (PDO) stent in vitro.

### 4.3 Results

The entire edge slide fastener PHB stents could release under the common pressure (11-15 atm) and lock successfully, and no stent curl toward intracavity; the diameter of stent is basically maintained at the predetermined diameter of lumen, the stents have very low acute elastic recoil rate (0.35%). The results prove that the stent design is feasible.

**Table 11 The results of the edge slide fastener PHB stents**

| | n | Releasing pressure (atm) | Diameter of lumen by stent expansion (mm) | Acute elastic recoil rate (%) | Successful locking ratio (%) |
|---|---|---|---|---|---|
| Edge slide fastener stent | 10 | 13±2 | 7.82±0.2 | 0.35±0.1 | 100 |

### 5. The release of edge slide fastener poly-L-lactic acid (PLLA) stent in vitro

### 5.1 Materials and methods:

Materials: ten edge slide fastener poly-L-lactic acid (PLLA) stents of 20x8 mm, rubber hose of 6 cm diameter, stent delivery system and force pump.

Methods: see also the release of snap fastener polydioxanone (PDO) stent in vitro.

### 5.2 Performance index

See also the release of snap fastener polydioxanone (PDO) stent in vitro.

### 5.3 Results

The entire edge slide fastener PLLA stents could release under the common pressure (11-15 atm) and lock successfully, and no stent curl toward intracavity; the diameter of stent is basically maintained at the predetermined diameter of lumen, the stents have very low acute elastic recoil rate (0.35%). The results prove that the stent design is feasible.

**Table 12 The results of the edge slide fastener PLLA stents**

| | n | Releasing pressure (atm) | Diameter of lumen by stent expansion (mm) | Acute elastic recoil rate (%) | Successful locking ratio (%) |
|---|---|---|---|---|---|
| Edge slide fastener stent | 10 | 13±2 | 7.82±0.2 | 0.35±0.1 | 100 |

The results of releases of five kinds of materials of edge slide fastener stents in vitro show that: all of five kinds of materials of edge slide fastener stents could release under the common pressure and lock successfully, and no stent curl toward intracavity; the diameters of stents are basically maintained at the predetermined diameter of lumen, the stents have very low acute elastic recoil rate. The edge slide fastener PDO stent has the lowest acute elastic recoil rate (0.3%), so PRO is the optimal material for producing edge slide fastener stent.

### Example 4

China Application of publication No. CN101484195A discloses a composite stent, this invention discloses a biodegradable or bioabsorbable multiple-layered or composite stent which includes a bioabsorbable ceramic material (like calcium phosphate, bioactive glass) coated with a biodegradable polymeric material (like polylactic acid (PLA), polylactide and/or polyglycolic acid (PGA), polyglycolide and/or poly (lactic-co-glycolic acid) (PLGA)). The disadvantages of the composite stent are that: the degradation rates of the PLA and PLGA materials are slow, complete degradation time is more than two years, the degradation rate of PGA is too fast, 70%-80% of PGA is degraded in two weeks, and the stent is easy to transform and slide and has an unsatisfactory supporting effect, bad bacteria resistance, easy to absorb moisture and be degraded in air, not easy to save, a small number of patients will have non-infectious inflammation, etc; the composite stent is made by multiple materials, more layers and complex production process; the quantity of stent clips of composite stent is small, therefore, the curly extent of the composite stent is limited thus the applicable range is limited; that the angle of stent clips is larger result in increasing resistance and release pressure and the risk of complication in the process of stent expansion. Meanwhile, the stent just has clips at the two sides and not the middle part, so the supporting force of the middle part decreases obviously.

Double fastener PDO stent: it is made by polydioxanone (PDO). The PDO fiber has advantages of good mechanical strength, chemical stability, biocompatibility, safety, biodegradability, easy processing and so on. The surface structure of PDO monofilament is smooth and continuous which overcome the shortcoming of fiber easy to damage organization because of big friction coefficient in the braided structure. PDO has good compatibility, mind tissue response, no cell reaction and safety because it can be degraded and absorbed gradually by organism by way of broken down into excreted carbon dioxide and water after 180 days. PDO is propitious to make various size of monofilament suture line because of its flexible molecular chain containing the ether bond. PDO has mind tissue reaction, and it is degraded by hydrolysis in vivo with a big strength retention rate, that is particularly useful for suturing the wound of longer healing time. From the view points of flexibility and big strength retention rate, PDO is the extremely suited macromolecular polymer for stent production.

The double fastener PDO stent is fabricated by a three-dimensional micro-jetting free molding technology. The predefined three-dimensional model is fabricated very accurately in accordance with the computer aided design with a flexible technology. The size of stent meshes, stent thickness and the length of stent clips and so on can be changed according to different requirements. The double fastener PDO stent has convenient drug carrying and simple production.

There are rows of even-distributed meshes of 1 mm in the body of double fastener PDO stent. The stent head consists of a framework; there are 2-5 clips in the framework. The two sides of stent body are equipped with barbs of 0.1 mm; all of the barbs make a 30 degrees angle relative to the lateral axis of stent and extend away from the stent head. The curly extent of stent can be adjusted according to the diameter of implanted position in stent usage, and thus the stent can be applicable to a wider range. The small angle of the barbs reduces the resistance of stent expansion and release pressure and the risk of complications. Meanwhile, the protruding clips located in the stent head can be inserted into the meshes to provide stronger and symmetrical supporting force.

In addition, the double fastener PDO stent can be equiped with a delivery system; hence the difficulty of surgery operation is reduced.

The delivery system consists of an outer cannula (3), an inner sheath (4) and a balloon catheter (5) as shown in Fig. 5. The outer cannula (3) consists of a proximal end, a distal end and the cavity among the two ends. The inner sheath (4) also consists of a proximal end, a distal end and the cavity among the two ends. The external diameter of inner sheath (4) is proper to insert in the cavity of the outer cannula (3), and the length of the inner sheath (4) is about 4-6cm longer than that of the outer cannula (3). The balloon catheter (5) also consists of a proximal end, a distal end and the cavity among the two ends. The external diameter of the balloon catheter (5) is proper to insert in the cavity of the inner sheath (4). The distal end of the balloon catheter (5) is a cone (52) and a balloon (51). The length and diameter of the balloon (51) can be chosen according to the requirement of stent. There is a metallic marker on each side of the balloon (51) which can position the stent. The delivery system also includes two Y-type adapters, one Y-type adapter (41) is mounted on the proximal end of the inner sheath (41) and connects its cavity, and another Y-type adapter (31) is mounted on the proximal end of the outer cannula (3) and connects its cavity. Y-type adapters are applied to inject and remove needed liquid in the delivery procedure of the slide fastener stent. The stent is coiled on the surface of balloon (51) and then fixed at the position among the cone (52) located at the distal end of balloon (51) and the inner sheath (4) located at the proximal end of balloon (51), and that could prevent stent from displacement. The stent can not unfold as it is limited in the outer cannula (3). The delivery device equipped with stent is sent to the narrow blood vessel by following a guide wire. When the delivery device is accurately located at destination under the action of metallic marker of balloon (51), the outer cannula (3) is withdrawn, and then the balloon (51) is inflated to allow for the release of the stent, subsequently, the stent adheres closely to the inner wall of blood vessel. Finally the inner sheath (4) and the outer cannula (3) are pulled out to finish stent implantation.

### Example 5 animal experiment of slide fastener bioabsorbable stent implantation

### 1. Materials and methods

Materials: forty one healthy pigs after the birth of 2-3 months (weighting 25-30kg, male or female, supplied by Agricultural College of Shanghai Jiao Tong University), forty five slide fastener PDO stents (6x20 mm, 0.28 mm thickness), delivery system.

Equipments: operation kits (Shanghai Children's Medical Center); 7F artery sheath, force pump, guide wire fed (Cordis Co., USA); puncture needle, angiographic catheter (Diag Co. USA); pressure capsule (S&W Medico Tekikls); GE LC/LP DSA (General Electric, USA); cardiogram monitor, breathing machine (Phillip Co.); microtome, microscope and image analytical system (Leica Co.); JSW-7401F field emission scanning electron microscope (JEOL Ltd., Japan).

### Methods:

□ Anesthesia: fasting in the day before surgery, pigs are anesthetized by 8-10mg/kg ketamine, intramuscular injection of atropine 0.02 mg/kg, and then establish venous access. After intravenous injected of chloride succinylcholine 2mg/kg, the pigs are immediately given endotracheal intubation, breathing machine auxiliary ventilation, electrocardiogram monitoring. Fentanyl 2µg/kg, ketamine 2mg/kg and vecuronium 0.1mg/kg are given discontinuously to maintain.
□ Fix: pigs are fixed on the heart catheter operating floor by special wooden shelf.
□ Routine sterilization, covered with sterile drape, separate left carotid artery, implant 7F artery sheath after punctured.
□ Angiography of the left and right iliac artery is performed by inserting 6F angiographic catheter, require the diameter ratio of the stent and the selected implanted blood vessel is 1.20-1.25: 1.
□ The balloon of delivery device is sucked into negative pressure by the force pump; the stent is coiled on the surface of the balloon when the outer cannula is withdrawn. Then the stent is enclosed when the outer cannula is pushed to the cone portion. The 7F artery sheath is withdrawn and the stent delivery device is delivered to the target portion by following a guide wire, and then the stent is dilated and released at 12 atmospheres for 30 s. The dosage of heparin is 200U/kg in surgery operation, if the operation time exceed 1 hour, add additional 2000U heparin.
□ After the wound is sutured and carried out with hemostasis by compression more than half an hour, pigs are send back to the breeding centre to continue breeding. Pigs are injected with cefazolin sodium 50mg/kg/day for three days, fraxiparine 5000U/day for five days and bamyl 5mg/kg/day until they are put to death.

### 2. Performance index

### 2.1 Success rate of implantation and Complication rate

Success rate of stent implantation, evaluation criterion: released and dilated successfully at target site, no stent fall off and shift, no vessel laceration, no hemorrhea, etc.

Complication rate: vessel laceration, hemorrhea, artery perforation, pig die and so on caused by the stent and delivery system.

### 2.2 Randomly efficacy assessments of bioabsorbable stent

Efficacy assessments of bioabsorbable stent are conducted at the immediate postoperative moment, 1 month and 3 months and 6 months post implantation. The diameter of stent is measured by cardiac catheter angiography.

The diameter of target blood vessel after stent implantation is the average value of the diameter of the proximal end, the middle portion and the distal end of the implanted stent by following computer measurement. The referenced diameter of blood vessel near the two ends of stent: the diameters of the blood vessels which are located at outside 0.5 cm of the two ends of stent, are all measured three times and calculated the average values, respectively; the average diameters of the two ends are added up and calculated average value again.

### 2.3 Biocompatibility and degradation rate of the stent

Pigs are put to death at 1 month and 3 months and 6 months post implantation. The organization of the two ends and middle and edge of the stent are carried out by HE staining to observe the inflammatory response of the inside of the stent and surrounding, the growth situation of granulation tissue, the growth situation of the endothelium of stent surface and the stent degradation situation.

### 2.4 The SEM and evaluation of the separated specimen after stent implantation

The stent together with the blood vessel organization of 0.5 mm length located at the two ends of stent are taken out and made into specimen, the extent of stent endothelialization is observed by SEM.

### 3. Results

### 3.1 General case

Forty one pigs and forty five stents are used in operation, two pigs appear accident due to anesthesia, and two pigs die due to hemorrhea caused by vessel laceration, two stents are not fully dilated, one stent is not fully released because the balloon rupture during implantation. Other pigs are in good condition, food intake normally, good mental state, move freely, no hemiplegia, no behavior abnormally, no diarrhea, no fever, no hematochezia, no macroscopic hematuria and so on.

### 3.2 The information features of the slide fastener stent implantation

The information features of the slide fastener stent implantation are shown in table 13. The success ratio of stent implantation is 88.90%, the success ratio of delivery system delivering is 93.30%, while the complication rate is 11.10%.

**Table 13 The information features of the slide fastener stent implantation**

| Variables | n (%) |
|---|---|
| Pig | 41 |
| Weight (kg) | 25.17±1.84 |
| Female | 16 (39%) |
| Male | 25 (61%) |
| Left iliac artery | 41 (91.10%) |
| Right iliac artery | 5 (8.90%) |
| Referenced diameter of blood vessel (mm) | 4.68±0.17 |
| Release pressure (atm) | 16.10±2.20 |
| Success ratio of implantation | 40 (88.90%) |
| Complication rate | 2 (4%) |
| Success ratio of delivery system | 42 (93.30%) |
| Failure ratio of implantation | 5(11.10%) |

### 3.3 Efficacy of the slide fastener stent

The diameter of target blood vessel is measured randomly after stent implantation as shown in table 14.

**Table 14 The variation of the diameter of target blood vessel post operation**

| | After implantation, n=9 | One month, n=9 | Three months, n=9 | Six months, n=9 |
|---|---|---|---|---|
| Diameter of blood vessel (mm) | 5.92±0.06 | 5.85±0.04 | 4.86±0.09*● | 4.84±0.11*● |

| | | | | |
|---|---|---|---|---|
| * compared with the value of after implantation P<0.01;• compared with the value of one months P<0.01. | | | | |

Table 13 shows that: the diameter of target blood vessel is unconspicuous change at the moment and one month after stent implantation (P>0.05, no statistical significant); but the diameter of lumen lose somewhat at three months and six months post operation, the lumen minish somewhat, the value compared with that of the moment and one month after implantation P<0.01, there is a observably statistical difference; but the diameter of lumen is unconspicuous change at three months and six months post implantation (P>0.05, no statistical significant).

### 3.4 Biocompatibility and degradability of stent

### □ Histocompatibility

One month post implantation: the bioabsorbable PRO stent is already covered with endothelial cells and still integrated and barely degraded; a few inflammatory cells mainly including leukomonocytes, plasmocytes and eosinophil granulocytes, begin to aggregate around the stent. Three months post implantation: the bioabsorbable PRO stent is covered with compact mature endothelial cells and damaged and partly degraded; there are still inflammatory cells mainly including leukomonocytes, eosinophil granulocytes and foreign body giant cells around the stent. Six months post implantation: the stent is mostly degraded; there are still a few inflammatory cells mainly including foreign body giant cells, leukomonocytes and plasmocytes aggregated around the stent; meanwhile the stented vessel gradually returned to the normal blood vessel along with the stent is degraded and absorbed and the inflammatory cells are reduced.

### □ Cytocompatibility

The bioabsorbable PRO stent is implanted into the iliac artery of pig, the observation with the naked eye show that: the surface of the stent is covered with tenuous intima at one month post implantation, covered with neointima totally at three months post implantation and covered with smooth glossy neointima at six months post implantation. The SEM show that: the stent is sparsely covered with endothelial cells at one month post implantation, compactly covered with endothelial cells at three months post implantation and covered with integrated intima at six months post implantation. The results prove the stent has good cytocompatibility.

### □ Degradability

The stent is still integrated and barely degraded at one month post implantation, damaged and partly degraded at three months post implantation, mostly degraded at six months post implantation. The results prove the stent has good degradability.

### 3.5 Conclusions

The slide fastener bioabsorbable PDO stent is successfully implanted into the iliac artery of pig by delivery system, which is technically feasible; the stent and delivery system have good success ratio and low complication rate, which is feasible in design; the PRO stent has a good efficacy in a short time (one month), the diameter of blood vessel lose somewhat at medium-term mainly result from endometrial hyperplasia, but the blood vessel still open well; the inflammatory cells begin to aggregate along with the stent degraded, but at the later period the inflammatory response disappear gradually along with the material is completely degraded; the PRO stent is fully covered with endothelial cells at one month post implantation, thus prove the stent has good cytocompatibility; the PRO stent is mostly degraded at six months post implantation, thus prove the stent has good degradability.

### Example 6 the application of the stent in the disease of coronary stenosis

### 1. Materials and methods

Materials: four miniature pigs weighting 18-20kg, target coronary vascular lumen of 2.2±0.2 mm diameter, four edge slide fastener PDO stents (20 mm length, 2.5-2.75 mm diameter), guide wire fed, stent delivery system and force pump.

### Methods:

□ Pigs are administered with oral aspirin 0.3g and clopidogrel 75mg, and anesthetized with intramuscular injection of atropine 0.02mg/kg and ketamine 10mg/kg.
□ Slice the skin after anesthesia, separate subcutaneous tissue, puncture after expose right femoral artery, implant 6F artery sheath, and give anticoagulant heparin sodium 200µ/kg. Angiography of the left and right iliac artery is performed by inserting 6F angiographic catheter, the diameter ratio of the stent and the selected implanted blood vessel is 1.10-1.20:1.
□ The balloon of the delivery system is sucked into negative pressure by the force pump; the stent is coiled on the surface of the balloon when the outer cannula is withdrawn, then the stent is enclosed when the outer cannula is pushed to the cone portion. After withdrawn the artery sheath, the stent delivery system is delivered to the target position (middle piece of anterior descending) by following a guide wire fed; the stent is dilated and released at 10-15 atmospheres for 15-20 s.
□ The balloon is sucked into negative pressure and after that the balloon is withdrawn; finally the delivery system is entirely withdrawn.

### 2. Performance index

□ The diameter of blood vessel lumen post operation: to measure the diameter of blood vessel lumen by stent expansion after the balloon is withdrawn.
□ Acute elastic recoil rate: the acute elastic recoil rate of stent = (the diameter of the fully expansive stent - the diameter of stent after the balloon is withdrawn) / the diameter of the fully expansive stent x 100%.
□ Successful locking ratio: the evaluation criteria are: success, the stent clips are locked after the balloon is withdrawn; failure, the stent clips are not locked after the balloon is withdrawn, the stent head curl inward.

### 3. Results

The efficacies of stents are showed in table 15.

**Table 15 The efficacies of the slide fastener stents**

| | n | Releasing pressure (atm) | Diameter of lumen by stent expansion (mm) | Acute elastic recoil rate (%) | Successful locking ratio (%) |
|---|---|---|---|---|---|
| PDO stent | 4 | 13±2 | 2.65±0.2 | 2±0.5 | 100 |

As shown in the table, the entire edge slide fastener PDO stents could release under the common pressure (10-15 atm) and lock successfully, and no stent curl toward intracavity; the diameters of stents are basically maintained at the predetermined diameter of lumen, the stents have very low acute elastic recoil rate (2%). The results prove that the stent design is feasible.

### Example 7 the application of the stent in the disease of peripheral vascular stenosis

### 1. Materials and methods

Materials: four healthy pigs after the birth of 2-3 months (weighting 25-30kg, male or female), target peripheral vascular lumen of 4.5±0.2 mm diameter, four double fastener PDO stents (20 mm length, 6 mm diameter), stent delivery system.

### Methods:

□ Anesthesia: fasting in the day before surgery, pigs are anesthetized by 8-10mg/kg ketamine, intramuscular injection of atropine 0.02 mg/kg, and then establish venous access. After intravenous injected of chloride succinylcholine 2mg/kg, the pigs are immediately given endotracheal intubation, breathing machine auxiliary ventilation, electrocardiogram monitoring. Fentanyl 2µg/kg, ketamine 2mg/kg and vecuronium 0.1mg/kg are given discontinuously to maintain.
□ Fix: pigs are fixed on the heart catheter operating floor by special wooden shelf.
□ Routine sterilization, covered with sterile drape, separate left carotid artery, implant 7F artery sheath after punctured.
□ Angiography of the peripheral blood vessel is performed by inserting 6F angiographic catheter, the diameter ratio of the stent and the selected implanted blood vessel is 1.20-1.25:1.
□ The balloon of delivery system is sucked into negative pressure by the force pump; the double fastener stent is coiled on the surface of the balloon when the outer cannula is withdrawn. Then the stent is enclosed when the outer cannula is pushed to the cone portion. The 7F artery sheath is withdrawn and the stent delivery syetem is delivered to the target portion by following a guide wire, and then the stent is dilated and released at 12 atmospheres for 30 s. The dosage of heparin is 200U/kg in operation, if the operation time exceed 1 hour, add additional 2000U heparin.
□ The balloon is sucked into negative pressure and after that the balloon is withdrawn; finally the delivery system is entirely withdrawn.

### 2. Performance index

□ The diameter of peripheral vascular lumen post operation: to measure the diameter of peripheral vascular lumen by stent expansion after the balloon is withdrawn.
□ Acute elastic recoil rate: the acute elastic recoil rate of stent = (the diameter of the fully expansive stent - the diameter of stent after the balloon is withdrawn) / the diameter of the fully expansive stent x 100%.
□ Successful locking ratio: the evaluation criteria are: success, the stent clips are locked after the balloon is withdrawn; failure, the stent clips are not locked after the balloon is withdrawn, the stent head curl inward.

### 3. Results

The efficacies of stents are showed in table 16.

**Table 16 The efficacies of the double fastener stents**

| | n | Releasing pressure (atm) | Diameter of lumen by stent expansion (mm) | Acute elastic recoil rate (%) | Successful locking ratio (%) |
|---|---|---|---|---|---|
| PDO stent | 4 | 12±2 | 5.9±0.2 | 2±0.5 | 100 |

As shown in the table, the entire double fastener PDO stents could release under the common pressure (10-14 atm) and lock successfully, and no stent curl toward intracavity; the diameters of stents are basically maintained at the predetermined diameter of lumen, the stents have very low acute elastic recoil rate (2%). The results prove that the stent design is feasible.

### Example 8 the application of the stent in the disease of esophagus

### stenosis

### 1. Modeling of experimental pigs of esophagus stenosis

Materials: four miniature pigs weighting 18-20kg, ketamine, atropine, diazepam, guide wire fed, urografin, balloon, force pump and GE-2005 angiography instrument.

### Specific methods:

1.1 Pigs are given intramuscular injection of atropine 0.02 mg/kg at preoperative 30 minutes, then anesthetized by 10mg/kg ketamine and fixed on the operating floor.
1.2 The restructured balloon catheter is inserted though mouth, the head of the balloon catheter is inserted into the middle of esophagus under X-ray. After balloon expansion, 1 ml 4% NaOH is injected into above the balloon; after 30 s, the gas of balloon is released, the water of 20 ml wash slowly for 1 min.
1.3 X-ray urografin angiography is performed after two weeks from modeling; the success criterin is that esophagus stenosis is more than 45%.

### 2. The edge slide fastener PRO stents are implanted into the esophageal lumen of the experimental pigs

### 2.1 Materials and methods

Materials: four edge slide fastener PRO stents (20 mm length, 8-12 mm diameter), guide wire fed, stent delivery system, force pump, four experimental pigs of esophagus stenosis, the original diameter of esophageal lumen of the experimental pigs is 10±2 mm, post modeling the diameter of the esophageal lumen is 6±0.5 mm.

### Methods:

□ Fasting during the 6 hours before surgery, pigs are anesthetized by intramuscular injection of atropine 0.02 mg/kg and ketamine 8-10mg/kg.
□ Urografin angiography is performed to determine the stenosis position, the length and diameter of stenosis segment with the purpose of selecting suitable stent.
□ Under X-ray observation, the delivery system equipped with slide fastener PDO stent is inserted into esophageal target site though mouth by following a guide wire fed, then the stent is dilated and released at 15 atmospheres for 30 s after accurately positioned.
□ The balloon is sucked into negative pressure and after that the balloon is withdrawn; finally the delivery system is entirely withdrawn by following guide wire fed.

### 2.2 Performance index

□ The diameter of esophageal lumen post operation: to measure the diameter of esophageal lumen by stent expansion after the balloon is withdrawn.
□ Acute elastic recoil rate: the acute elastic recoil rate of stent = (the diameter of the fully expansive stent - the diameter of stent after the balloon is withdrawn) / the diameter of the fully expansive stent x 100%.
□ Successful locking ratio: the evaluation criteria are: success, the stent clips are locked after the balloon is withdrawn; failure, the stent clips are not locked after the balloon is withdrawn, the stent head curl inward.

### 2.3 Results

The efficacies of stents are showed in table 17.

**Table 17 The efficacies of the edge slide fastener stents**

| | n | Releasing pressure (atm) | Diameter of lumen by stent expansion (mm) | Acute elastic recoil rate (%) | Successful locking ratio (%) |
|---|---|---|---|---|---|
| PDO stent | 4 | 15±2 | 9.5±0.5 | 2±0.5 | 100 |

As shown in the table, the entire edge slide fastener PDO stents could release under the common pressure (13-17 atm) and lock successfully, and no stent curl toward intracavity; the diameters of stents are basically maintained at the predetermined diameter of lumen, the stents have very low acute elastic recoil rate (2%). The results prove that the stent design is feasible.

### Example 9 the application of the stent in the disease of trachea stenosis

### 1. Modeling of experimental pigs of trachea stenosis

□ Fasting during the 6 hours before surgery, pigs are anesthetized by intramuscular injection of atropine 0.02 mg/kg and ketamine 10mg/kg at preoperative 30 minutes, then fixed on the operating floor.
□ Separate the skin and subcutaneous layer by layer, expose the trachea, adopt the surgical local excision joint legal to make the trachea stenosis more than 45%, then suture layer by layer.

### 2. The double fastener PDO stents are implanted into the tracheal lumen of the experimental pigs

### 2.1 Materials and methods

Materials: four double fastener PRO stents (20 mm length, 15 mm diameter), multi-purpose catheter, guide wire fed, stent delivery system, force pump, four experimental pigs of trachea stenosis, the original diameter of tracheal lumen of the experimental pigs is 14±1.5 mm, post modeling the diameter of the tracheal lumen is 7±1 mm.

### Methods:

□ Preoperative chest radiography and chest CT, airway 3D reconstruction, bronchoscopy are performed to make a preliminary understanding of the stenosis position and scope, the length and diameter of stenosis segment with the purpose of selecting suitable stent.
□ Pigs are given hypodermic injection of 0.5mg atropine to reduce respiratory tract secretions. 4% lidocaine is sprayed to the throat to make local anesthesia, a fibrous bronchoscope is inserted though the nose or mouth. 2% lidocaine and 1% epinephrine 2ml are injected to the trachea to make local anesthesia and contract the blood vessel of trachea in order to reduce cough response and bleeding in surgery.
□ Under the guide of X-ray, the multi-purpose catheter cooperated with smooth guide wire fed are inserted in trachea though glottis, afterwards, the fortified metallic guide wire fed replace the smooth guide wire fed and pass over the stenosis segment by following the catheter. The guide wire fed is left in the distal end of the stenosis segment, the catheter is withdrawn.
□ The delivery system equipped with stent is inserted to stenosis segment by following a guide wire fed, and then the stent is dilated and released at 14 atmospheres for 30 s. The balloon is sucked into negative pressure and after that the balloon is withdrawn; finally the delivery system is entirely withdrawn by following the guide wire fed.

### 2.2 Performance index

□ The diameter of tracheal lumen of the pig: to measure the diameter of tracheal lumen by stent expansion after the balloon is withdrawn.
□ Acute elastic recoil rate: the acute elastic recoil rate of stent = (the diameter of the fully expansive stent - the diameter of stent after the balloon is withdrawn) / the diameter of the fully expansive stent x 100%.
□ Successful locking ratio: the evaluation criteria are: success, the stent clips are locked after the balloon is withdrawn; failure, the stent clips are not locked after the balloon is withdrawn, the stent head curl inward.

### 2.3 Results

The efficacies of stents are showed in table 18.

**Table 18 The efficacies of the double fastener stents**

| | n | Releasing pressure (atm) | Diameter of lumen by stent expansion (mm) | Acute elastic recoil rate (%) | Successful locking ratio (%) |
|---|---|---|---|---|---|
| PDO stent | 4 | 14±2 | 12.75±1.25 | 3±0.7 | 100 |

As shown in the table, the entire double fastener PDO stents could release under the common pressure (12-14 atm) and lock successfully, and no stent curl toward intracavity; the diameters of stents are basically maintained at the predetermined diameter of lumen, the stents have very low acute elastic recoil rate (3%). The results prove that the stent design is feasible.

### Example 1 the application of the stent in the disease of biliary tract stenosis

### 1. Modeling of experimental pigs of biliary tract stenosis

□ Fasting during the 6 hours before surgery, pigs are anesthetized by intramuscular injection of atropine 0.02 mg/kg and ketamine 10mg/kg at preoperative 30 minutes, then fixed on the operating floor.
□ The curved incision is performed at the right upper abdomen and under costal margin to isolate common bile duct. Adopt the local sewing shrink method to make the common bile duct stenosis more than 50%, then suture abdomen layer by layer. The conventional treatment with antibiotics is carried out after surgery.

### 2. The middle slide fastener PRO stents are implanted into the biliary tract lumen of the experimental pigs

### 2.1 Materials and methods

Materials: four middle slide fastener PRO stents (25 mm length, 6-8 mm diameter), rubber hose, stent delivery system, force pump, four experimental pigs of biliary tract stenosis, the original diameter of biliary tract lumen of the experimental pigs is 7.5±0.5 mm, post modeling the diameter of the biliary tract lumen is 4±0.3 mm.

### Methods:

□ Preoperative endoscopic retrograde cholangiopancreatography is performed with duodenoscope to ascertain the property and scope of biliary tract stenosis, the diameter and length of stenosis segment with the purpose of selecting suitable stent.
□ Fasting during the 6 hours before surgery, pigs are given intramuscular injection of anisodamine 10mg, pethidine 20mg and diazepam 10mg at preoperative 30 minutes to reduce peristalsis and to make duodenum in nervous state to facilitate the operation.
□ Endoscopic retrograde cholangiopancreatography is performed with duodenoscope to ascertain the stenosis position of biliary tract. The guide wire fed is inserted to left hepatic duct or right hepatic duct though angiographic catheter, the stent delivery system is inserted to target site of biliary tract by following a guide wire fed, and then the stent is dilated and released at 12-14 atmospheres for 30 s.
□ The balloon is sucked into negative pressure and after that the balloon is withdrawn; finally the delivery system is entirely withdrawn by following the guide wire fed.

### 2.2 Performance index

□ The diameter of biliary tract lumen of the pig: measure the diameter of biliary tract lumen by stent expansion after the balloon is withdrawn.
□ Acute elastic recoil rate: the acute elastic recoil rate of stent = (the diameter of the fully expansive stent - the diameter of stent after the balloon is withdrawn) / the diameter of the fully expansive stent x100%.
□ Successful locking ratio: the evaluation criteria are: success, the stent clips are locked after the balloon is withdrawn; failure, the stent clips are not locked after the balloon is withdrawn, the stent head curl inward.

### 2.3 Results

The efficacies of stents are showed in table 19.

**Table 19 The efficacies of the middle slide fastener stents**

| | n | Releasing pressure (atm) | Diameter of lumen by stent expansion (mm) | Acute elastic recoil rate (%) | Successful locking ratio (%) |
|---|---|---|---|---|---|
| PDO stent | 4 | 12±2 | 7.5±0.5 | 3±0.5 | 100 |

As shown in the table, the entire middle slide fastener PDO stents could release under the common pressure (12-14 atm) and lock successfully, and no stent curl toward intracavity; the diameters of stents are basically maintained at the predetermined diameter of lumen, the stents have very low acute elastic recoil rate (3%). The results prove that the stent design is feasible.

### Example 11 the application of the stent in the disease of urethra stenosis

### 1. Modeling of experimental dogs of urethra stenosis

□ Fasting during the 6 hours before surgery, dogs are anesthetized by intramuscular injection of atropine 0.02 mg/kg and ketamine 10mg/kg at preoperative 30 minutes, then fixed on the operating floor.
□ Prepuce of penis is incised from abdomen side to fully expose external orifice of urethra. F6 catheter is inserted from external orifice of urethra and left at 1cm depth. Retrograde urethrography is performed by urografin which concentration of 760 g/L is diluted to 150 g/L using normal saline. 10F pediatric electric cutting mirror is inserted to the depth of 5-6 cm. Electrocision is performed by the cyclic electrode of 2 mm diameter under conditions of 30W electric cutting power, washing fluid of 5% glucose, at 5-7 point position of urethrascope view, to make urethra stenosis more than 50%, then suture abdomen layer by layer. The conventional treatment with antibiotics is carried out after surgery.

### 2. The edge slide fastener PDO stents are implanted into the urethral lumen of the experimental dogs

### 2.1 Materials and methods

Materials: four edge slide fastener PDO stents (20 mm length, 10 mm diameter), guide wire fed, rubber hose, stent delivery system, force pump, four experimental dogs of urethra stenosis, the original diameter of urethral lumen of the experimental dogs is 10-12 mm, post modeling the diameter of the urethral lumen is 5-6 mm.

### Methods:

① Surface anesthesia of urinary tract mucosa is performed by injection of 5ml 1% lidocaine from the external orifice of urethra before surgery. Urethrography is carried out by injection of contrast agent under the guidance of DSA, to ascertain the stenosis position, the diameter and length of stenosis segment with the purpose of selecting suitable stent.
② Preoperative treatment with antibiotics for 3-5 days, urinary tract mucosa is anesthetized by 1% lidocaine. The guide wire fed is inserted into bladder though urethra under the guidance of DSA. According to the results of the urinary tract radiography, the stent delivery system is inserted to target site of urinary tract by following a guide wire fed, and then the stent is dilated and released at 12 atmospheres for 30 s.
③ The balloon is sucked into negative pressure and after that the balloon is withdrawn; finally the delivery system is entirely withdrawn by following the guide wire fed.

### 2.2 Performance index

① The diameter of urethral lumen of the dog: measure the diameter of urethral lumen by stent expansion after the balloon is withdrawn.
② Acute elastic recoil rate: the acute elastic recoil rate of stent = (the diameter of the fully expansive stent - the diameter of stent after the balloon is withdrawn) / the diameter of the fully expansive stent ×100%.
③ Successful locking ratio: the evaluation criteria are: success, the stent clips are locked after the balloon is withdrawn; failure, the stent clips are not locked after the balloon is withdrawn, the stent head curl inward.

### 2.3 Results

The efficacies of stents are showed in table 20.

**Table 20 The efficacies of the edge slide fastener stents**

| | n | Releasing pressure (atm) | Diameter of lumen by stent expansion (mm) | Acute elastic recoil rate (%) | Successful locking ratio (%) |
|---|---|---|---|---|---|
| PDO stent | 4 | 12±2 | 10±2 | 5±1 | 100 |

As shown in the table, the entire edge slide fastener PDO stents could release under the common pressure (10-14 atm) and lock successfully, and no stent curl toward intracavity; the diameters of stents are basically maintained at the predetermined diameter of lumen, the stents have very low acute elastic recoil rate (5%). The results prove that the stent design is feasible.

### Example 12 the application of the stent in the disease of intestinal tract stenosis

### 1. Modeling of experimental pigs of intestinal tract stenosis

### ① Fasting during the 6 hours before surgery, pigs are anesthetized by intramuscular injection of atropine 0.02 mg/kg and ketamine 10mg/kg at preoperative 30 minutes, then fixed on the operating floor.

□ The incision is performed at the left abdomen to isolate descending colon. Adopt the local sewing shrink method to make the descending colon stenosis more than 50%, then suture abdomen layer by layer. The conventional treatment with antibiotics is carried out after surgery.

### 2. The middle slide fastener PDO stents are implanted into the intestinal tract lumen of the experimental pigs

### 2.1 Materials and methods

Materials: four middle slide fastener PDO stents (40 mm length, 20 mm diameter), rubber hose, guide wire fed, stent delivery system, force pump, four experimental pigs of intestinal tract stenosis, the original diameter of intestinal tract lumen of the experimental pigs is 20±2 mm, post modeling the diameter of the intestinal tract lumen is 10±2 mm.

### Methods:

□ Pigs are administered with conventional injection of 10mg 654-2 and 10mg diazepam. Under the direct vision of colonoscopy, the smooth guide wire fed is inserted into the distal end of colon though the stenosis segment of colon. Double-lumen catheter is introduced along the guide wire. Under X-ray observation, radiography of injection of 60% urografin is performed to observe the condition of stenosis segment with the purpose of selecting suitable stent.
□ The catheter is further inserted deep into the distal end of stenosis segment, and exchange the soft head super-hard guide wire. Under X-ray observation, the stent delivery system is delivered to the target position of intestinal tract by following the guide wire fed; the stent is dilated and released at 12-14 atmospheres for 30 s.
□ The balloon is sucked into negative pressure and after that the balloon is withdrawn; finally the delivery system is entirely withdrawn.

### 2.2 Performance index

□ The diameter of intestinal tract lumen post operation: measure the diameter of intestinal tract lumen by stent expansion after the balloon is withdrawn.
□ Acute elastic recoil rate: the acute elastic recoil rate of stent = (the diameter of the fully expansive stent - the diameter of stent after the balloon is withdrawn) / the diameter of the fully expansive stent ×100%.
□ Successful locking ratio: the evaluation criteria are: success, the stent clips are locked after the balloon is withdrawn; failure, the stent clips are not locked after the balloon is withdrawn, the stent head curl inward.

### 2.3 Results

The efficacies of stents are showed in table 21.

**Table 21 The efficacies of the middle slide fastener stents**

| | n | Releasing pressure (atm) | Diameter of lumen by stent expansion (mm) | Acute elastic recoil rate (%) | Successful locking ratio (%) |
|---|---|---|---|---|---|
| PDO stent | 4 | 12±2 | 18±2 | 5±2 | 100 |

As shown in the table, the entire middle slide fastener PDO stents could release under the common pressure (10-14 atm) and lock successfully, and no stent curl toward intracavity; the diameters of stents are basically maintained at the predetermined diameter of lumen, the stents have very low acute elastic recoil rate (5%). The results prove that the stent design is feasible.

The aforementioned documents are preferred embodiments of this invention. It should be pointed out that the invention can be make a number of improvements and additions at the precondition of not separate from the present invention method for the common technical personnel of the technology field, and these improvements and additions should be considered in the scope of protection of this present invention.

## Claims

1. Use of a slide fastener bioabsorbable stent as a cardiovascular system stent or a lumen stent in the disease of cardiovascular or narrow lumen.

2. The use of slide fastener bioabsorbable stent according to claim 1, wherein said disease of narrow cardiovascular system is coronary artery stenosis, carotid artery stenosis, renal artery stenosis, pulmonary artery and its branches stenosis, aorta and its branches stenosis, cardinal vein stenosis or pulmonary vein stenosis.

3. The use of slide fastener bioabsorbable stent according to claim 1, wherein said disease of narrow lumen is trachea stenosis, esophagus stenosis, biliary tract stenosis, urethra stenosis or intestinal tract stenosis.

4. The use of slide fastener bioabsorbable stent according to claim 1, wherein said stent comprising:
an applanate stent body with mesh structure,
a stent head, which is located at one end of said stent body, said stent head is integrated with said stent body with matched size, and which provides a slide fastener effect when the stent is being curled,
stent clips, which are integrated with said stent body and fix the tubular structure of the stent when it is being curled.

5. The use of slide fastener bioabsorbable stent according to claim 1, wherein said stent material is polydioxanone (PDO), polylactic acid (PLA), polycaprolactone (PCL), polyglycolic acid (PGA) or polyhydroxybutyrate (PHB) macromolecular polymer.

6. The use of slide fastener bioabsorbable stent according to claim 5, wherein said stent material is polydioxanone (PDO).

7. The use of slide fastener bioabsorbable stent according to claim 4, wherein said stent also includes a delivery device, said delivery device comprising:
an outer cannula, consisting of a proximal end, a distal end and the cavity among the two ends, and
an inner sheath, consisting of a proximal end, a distal end and the cavity among the two ends, the external diameter of said inner sheath is proper to insert in the cavity of said outer cannula, and
a balloon catheter, consisting of a proximal end, a distal end and the cavity among the two ends, the external diameter of said balloon catheter is proper to insert in the cavity of said inner sheath, there is a balloon in the distal end of said balloon catheter, the lamellar slide fastener stent can be coiled on the surface of said balloon and subsequently delivered to the narrow lumen through the catheter.

8. A new bioabsorbable stent, wherein said stent includes a snap fastener stent, an edge slide fastener stent, a middle slide fastener stent and a double fastener stent, wherein said double fastener stent comprising:
an applanate stent body with mesh structure,
a stent head, which is located at one end of said stent body, said stent head is integrated with said stent body with matched size, and which provides a slide fastener effect when the stent is being curled,
stent clips, which are integrated with said stent body and consist of barbs located at the two sides of said stent body and stent head clips located at said stent head, and which fix the tubular structure of the stent when it is being curled.
